# EUROPEAN PATENT SPECIFICATION

(11) **EP 0 509 120 B1**
(45) Date of publication and mention of the grant of the patent: **17.07.1996**
(21) Application number: 91106227.1
(22) Date of filing: 18.04.1991
(51) Int. Cl.: A61K 31/725, A61K 31/73

(54) **Topical use of glycosaminoglycans in the prevention and treatment of diseases affecting the cervical vaginal tract**
Topische Anwendung von Glycosaminoglykane zur Prävention und Behandlung der Zervikal- oder Vaginalerkrankungen
Utilisation topique de glycosaminoglycanes pour la prévention et le traitement des maladies du tractus cervicovaginal

(43) Date of publication of application: 21.10.1992
(73) Proprietor: LABORATORI BALDACCI Spa, 56100 Pisa (IT)
(72) Inventor: Baldacci, Massimo, I-56100 Pisa (IT); Comi, Roberto, I-50100 Firenze (IT); Comi, Giovanna, I-50100 Firenze (IT)
(74) Representative: Dragotti, Gianfranco

(56) References cited:
- WO-A-89/05645
- DE-A- 3 441 835
- US-A- 4 486 416
- US-A- 4 711 780
- BAILLIERE'S CLINICAL OBSTETRICS AND GYNAECOLOGY, vol. 4, no. 2, June 1990, pages 263-282, Baillière Tindall; N. ULDBJERG et al.: "The physiology of cervical ripening and cervical dilatation and the effect of abortifacient drugs"
- BR. J. CANCER, vol. 57, no. 1, 1988, pages 74-78, The Macmillan Press Ltd; T. FUKATSU et al.: "Immunohistochemical localization of chondroitin sulphate and dermatan sulphate proteoglycans in tumour tissues"
- JOURNAL OF CELLULAR PHYSIOLOGY, vol. 125, no. 3, 1985, pages 499-506, Alan R. Liss, Inc.; T.C. WRIGHT, Jr. et al.: "Inhibition of rat cervical epithelial cell growth by heparin and its reversal by EGF"

## Description

The present invention relates to the use of glycosaminoglycans, as the only active ingredient for the preparation of a topical pharmaceutical composition for the prevention and therapy of diseases of the cervical vaginal area. As it is well known, the glycosaminoglycans (GAG) belong to a highly heterogeneous class of glucidic macromolecules of relevant biological interest.They comprise very long molecules containing repeating disaccharide units bond together to form linear macromolecules.

Each of these repeating units comprises a residue consisting of an aminosugar (glucosamine or galactosamine) and a residue usually consisting of uronic acid (or glucuronic acid). The hydroxyl group at C₄ or C₆ is generally esterified by sulfur containing groups.

The GAGs, owing to the fact of possessing always an acidic group per each disaccharide unit, have in solution a behaviour like that of anionic polyelectrolytes.

The GAGs are normally combined by a covalent bond to proteins and form therewith great molecules.There are thus formed the complexes called proteoglycanes. As it is known, the GAGs are represented by the following compounds:heparin, heparan sulfate, dermatan sulfate, hyaluronic acid, chondroitin sulfate A+C, keratan sulfate.

The GAGs belong to the matrix or fundamental substance of the connective tissue and thanks to their action this substance keeps the gel status which is essential for the function thereof.

The GAGs have relevant importance in order to control the water content at the tissue level, since water is retained from the GAG molecules and only in a minimum proportion is present in free form.

The GAGs, being expanded in their seat, form a sort of tridimensional network occupying a volume much greater than that of their molecular components.

This network is soaked by water and solutes (electrolytes). Also the water coming from the blood capillaries and taking part in the forming of the tissue liquid (through which the exchanges between blood and tissues and viceversa occur) is contained in the same network.

Important properties of the connective tissue mostly depend on the GAGs, such as for example the resistance to the tensile and compression stresses, the oxygen and substrate transport, the processes of selective filtration.

The GAGs take actively part in the processes of restoring and healing of the tissues and are thus effective agents in the recovery process of wounds and lesions.

This action is related to the GAGs activity in the promotion of the cell growt and proliferation as well as in the process itself of cell differentiation.

In the recovery processes of wounds and of substance losses as well as in the tissue proliferation a local concentration of GAGs occurs which takes the maximum values in the first days of the restoring process (Sodeman W.A., Fisiopatologia, ed.Piccin, 1978).

The GAGs are strictly connected to the collagen and elastic fibers and have a direct action on their forming.Thus the GAGs take active part in the forming and stabilizing of the collagen and elastic fibers. It has been found that GAGs have a relevant anticoagulating, antithrombotic and profibrinolytic action.

The GAGs activate the clearing factor and have therefore a lipolytic action.

An antiphlogistic and immunosuppressing action has been found for the GAGs (G.Segre, Min.Med, 77,1859, 1986).

As the years go (middle-aged to old persons) the biological supply of the GAGs becomes insufficient, whereby their availability,especially in the women, is reduced.

As a consequence of their relevant biological activity, GAGs have been already widespread use in the therapeutical field.
a) Use by systemic way (oral and parenteral). GAGs have been employed especially in the circulatory field for the treatment of vasculopathies having atherosclerotic basis and related forming of thrombi and lipids and of thrombus forming vein-pathologies.
b) Use by local route.

GAGs have been used by local route in form of gels, creams, pommades, medicated gauze, etc., owing to their various properties: antioedema, antitranspiration, anticoagulation and antiphlogistic, restoring and healing.

A number of preparations exists for the treatment of vein diseases at the skin surface (thrombophlebitis, varicophlebitis), of inflammatory situations,of haematomas and haemorrhagic bleedings.

A field of local use is that of the activating treatment of the tissue recovery processes, especially those having slow and torpid course: stasis induced ulcers (varicose and post-phlebitis ulcers)), trophic bed-sores.

Not negligible references as regards the presence of GAGs in the cervical vaginal district and on the role they have in this area are found in the literature.

The cervical epithelium (cylindrical epithelium coating the mucus secreting cervical channel) is devoid of glycogen, but has an abundant content of glycosaminoglycans (Delle Piane G., Maurizio E. and Tesauro G., Trattato Italiano di Ginecologia, Ed. P.E.M., Roma, 1972). The GAG fraction is 18% of the non gravidic uterine neck tissue and it continuously increases during the pregnancy (Kondot et al, Nagoya Journal of Med.Sc., 17, 203, 1971).

Hyaluronic acid, dermatan sulfate and chondroitin-4- and -6- sulfates are present in the cervix uteri (Danforth D.N. et al, Am. J. Obstetr. and Gynecol., 120, 641, 1974). Von Maillot et all, Am. J. Obstetr. Gynec., 220. 275, 1976, have measured by means of a quantitative chromatographic method the GAGs obtained by biopsy from the human cervix uteri and have found that chondroitin-4- and -6- sulfates and the dermatan sulfate represent 55% of the GAG content of the cervix uteri itself in the non pregnant state.

The hyaluronic acid is contained in an amount of 22%.

At the first birth step the content of dermatan sulfate and chondroitin sulfate is lowered to 31% and after the birth to 29%; the hyaluronic acid during the birth can be associated with the increase of the deformability of the cervix tissue (Von Maillot et al, Arch. fuer Gynakologie, 223,323-331,1977).

The modifications of the GAG distribution in the cervix during the pregnancy might modify not only the synthesis but also the catabolism of the collagen (Von Maillot et al, Am.J.Obstetr. and Gynec., 135, 503 1979). A high concentration of GAGs is present in the ovary, uterus, cervix uteri and vagina (Stephens H et al, Comp. Biocem. Physiol., 84,29,1986).82% of the material consisting of glycopeptides and of GAGs as isolated from the cervix uteri comes from the cervical mucus (id.).

From the human cervix uteri glycosaminoglycans have been isolated. Most of the material comes from the cervical mucin: it contains 3% hyaluronic acid, 13% dermatan sulfate, 2% heparan sulfate (Uldbjerg N. et al., Am.J.Obst.Gynecol. 16/4/199 - 1983). The making up of the reduced collagen by GAGs in the cervical area (such as that resulting from the local application of prostaglandin E₂ and increase of the collagenase) may be important in the recovery process (Uldbjerg N. et al., Am.J.Obst.Gynecol., 147, 1983).

GAGs modulate the growth and the recovery of the epithelium cells of the cervix uteri, which may be important for the control of the proliferation of these cells under normal and pathological conditions (Wright, J.Cell.Phys., 125(3)499- 1985).

The cervical mucus has a high GAG content. The vagina too has a high GAG content (Polonowski M., Biochimie Medicale, Masson ed., 1972; Stephens H. et al., Comp.Biochem.Physiol., 84, 29, 1986).

As regards the relationship between GAG and cervical vaginal area two features are of major importance: the forming and function of the mucus and the vaginal ecosystem.

GAGs have seemingly essential importance for the forming and the function of the cervical vaginal mucus.

This fact has particular importance since the mucus has, as it is well known, a protective and defensive role owing to the physical properties thereof, such as adhesivity, viscosity and gel formation; mucus moreover has a generical antibacterial activity.

It is further to be considered the intervention of the GAGs for the maintaining and the restoring of the water content of the mucus, owing to the relevant capability of GAGs of absorbing and retaining water and owing to the relevant water content of the mucus itself.The mucus of the cervical channel for example contains 92 to 94% of water in the phases before and after the surgical intervention and 98% at the middle time of the cycle (Polonowski M., Biochemie Medicale, Masson ed., 1972).

The haigh GAG content of the vagina is well adapted to and by itself takes part in the safeguarding and restoring of the natural vaginal ecosystem.

The vagina with its mucus has a relevant acidity, which is maximum at the ovulation time (oH = 3).

Under normal conditions the secretion is definitely acidic (pH of between 3.5 and 5). For this acidity the presence is important of lactic acid derived from the glycogen of the cells peeled off the stratified squamous epithelium of the vagina, having a high glycogen content.

GAGs too, which are present in a high content in the vagina with the mucus, must be taken into the due account, owing to their acidic character.

It has been now found and is the object of the present invention that the local application of GAGs, both as a mixture and in form of single components, is therapeutically advantageous and affords a prevention activity in the gynecological field and more specifically in the cases of cervical vaginal pathology in climacteric and post-climacteric condition, of vaginal pathology induced from various lesive agents or from drugs, and of aspecific vaginal pathology.

More specifically the present invention is directed to the topical use of glycosaminoglycans either singly or in admixture and selected among heparan sulfate, dermatan sulfate, chondroitin sulfates A+C and hyaluronic acid, in the prevention and treatment of states of:
dystrophy and atrophy due to lack of estrogenic hormones and senile vaginitis;
compromission and damage of the vaginal ecosystem induced by lesive agents, in the vaginal dermatitis and vaginitis;
so-called aspecific vaginitis, and
aspecific cervicitis with subacute course and tending to chronic course, said use consisting in the topical application of a composition selected among ovuli, creams, pommades, lotions and ointments containing the active ingredient at a concentration of between 0.5 and 5% .

As it will be seen from the experimental data as hereinafter reported and without aiming to limit the scope of the present invention, it seems reasonable to possibly attribute the advantageous results, obtained both in the case of therapy and in that of the prevention, to the purposely induced supply of glycosaminoglycans in the cervical vaginal area, suffering from a number of damage conditions and of structural and functional compromission thereof.

This supply seems to be able to have two effects:
a) a first effect in which the local status of compromission and deficit of GAGs is substituted for and compensated avoiding the related lesive consequences;
b) a symptomatic and pharmacodynamic effect adapted to bring in the cervical vaginal area the whole of activity and effects characteristic of the mechanism of action of GAGs, such as the intervention in the adjustment of the haemato-vascular and vascular tissue ratios, the intervention on the trophism and the function of the connective tissue and on the state of tissue hydration, the intervention with anticoagulating, antiphlogistic, antitranspiration effect and the intervention having trophic and recovery value.

These two effects (a and b) may coexist.

It is further to be pointed out, and that holds true both for the substitution effect and for the symptomatic effect, that the supply of GAGs takes place with a physiological mechanism, the natural state of morphological cervical vaginal functionality and particulalrly the normal condition of the vaginal ecosystem being fully respected (or restored).

Such a property, to be considered an almost exclusive characteristic of the GAG supply, is of particular interest for the treatment of the aspecific vaginitis, since it is well known that the respect of the vaginal ecosystem represents by itself the most effective defense mechanism against the arising of infections in this area and against their relapsing.

In the topical applications as above taken into consideration there are preferred compositions of the above defined type, containing 1% of GAG, with a posology foreseeing 1 to 2 daily applications for 1 to 2 weeks as an attack treatment and a subsequent phase for a time of 1 to 2 months in which a single dose is applied either daily or every two days.

In the following tables the data are reported of the experimental investigations relating to the topical use of GAGs under the conditions of the present invention, taking it into account that the attributed score is indicated by (+) and that (+++) represents an optimum therapeutical result and (++) a good result.

Moreover the useful effect of the topical treatment has been detected in almost all cases (90 to 95%) in all types of use.

The GAGs used were the following: heparan sulfate, dermatan sulfate, chondroitin sulfate A, chondroitin sulfate C, hyaluronic acid, both alone and as mixtures of two or more components. Examples of the latter are the following:
heparan sulfate + dermatan sulfate
dermatan sulfate + CSA
dermatan sulfate + hyaluronic acid

**TABLE 1**

| Cervical vaginal pathology in climacteric and post climacteric state, senile vaginitis (50 cases) | | | | |
|---|---|---|---|---|
| Active compound | obtained therapeutical results | | | remarks |
| | +++ | ++ | + | |
| DERMATAN sulfate | 95% | 5% | | optimum tollerance* |
| HEPARAN sulfate | 92% | 8% | | optimum tollerance* |
| CHONDROITIN sulfate A | 94% | 6% | | optimum tollerance* |
| CHONDROITIN sulfate B | 86% | 14% | | optimum tollerance* |
| HYALURONIC acid | 92% | 8% | | optimum tollerance* |

Evident eutrophic and revivifying effect on the dystrophy and atrophy state. Regression and disappearance of the subjective symptomatology such as burning, itch, pain, dryness feeling, dispareunia.

Definite benefit on the psycho-emotional sphere. Improvement and disappearance of the simptoms of the senile vaginitis.

**TABLE 2**

| Compromission and damaging of the vaginal ecosystem from lesive agents iatrogenic vaginitis (38 cases) | | | | |
|---|---|---|---|---|
| Active compound | obtained therapeutical results | | | remarks |
| | +++ | ++ | + | |
| DERMATAN sulfate | 95% | 5% | | optimum tollerance |
| HEPARAN sulfate | 88% | 12% | | optimum tollerance |
| CHONDROITIN sulfate A | 92% | 8% | | optimum tollerance |
| CHONDROITIN sulfate B | 85% | 15% | | optimum tollerance |
| HYALURONIC acid | 90% | 10% | | optimum tollerance |

Evident and remarkable eutrophic and revivifying effect on the vaginal ecosystem with reduction and disappearance of the objective symptoms (for example of the lesions of erythematic-bullate or vesicle-bullate type and of the related erosion features) as well as reduction and disappearance of the subjective symptoms:burning feeling, itch,pain.

**TABLE 3**

| Aspecific vaginitis (40 cases) | | | | |
|---|---|---|---|---|
| Active compound | obtained therapeutical results | | | remarks |
| | +++ | ++ | + | |
| DERMATAN sulfate | 96% | 4% | | optimum tollerance |
| HEPARAN sulfate | 90% | 10% | | optimum tollerance |
| CHONDROITIN sulfate A | 92% | 8% | | optimum tollerance |
| CHONDROITIN sulfate B | 82% | 18% | | optimum tollerance |
| HYALURONIC acid | 90% | 10% | | optimum tollerance |

Regression and disappearance of the phlogosis and restoring of the normal vaginal acidity and of the growth of lacto-bacilli. Definite reduction and ready disappearance of the bad smelling vaginal leaks and of the leukorrhea as well as ready reduction and disappearance of the subjective symptoms (itch, burning, pain, trouble feeling, dispareunia etc.).

**TABLE 4**

| Subacute cervicitis tending to chronicize erosions cervical ulcers (35 cases) | | | | |
|---|---|---|---|---|
| Active compound | obtained therapeutical results | | | remarks |
| | +++ | ++ | + | |
| DERMATAN sulfate | 94% | 6% | | optimum tollerance |
| HEPARAN sulfate | 88% | 12% | | optimum tollerance |
| CHONDROITIN sulfate A | 90% | 10% | | optimum tollerance |
| CHONDROITIN sulfate B | 80% | 20% | | optimum tollerance |
| HYALURONIC acid | 92% | 8% | | optimum tollerance |
| Note:Remarkable healing of the lesions almost constantly obtained Evident reduction up to disappearance of the inflammatory state as well as regression and restoring of the related leaks of substance of ulcering and erosive type. | | | | |

## Claims

1. Use of glycosaminoglycans, both singly and in admixture, as the only active ingredient for the preparation of a topical pharmaceutical composition for the prevention and therapy of diseases of the cervical vaginal area.

2. Use according to claim 1, characterized in that said glycosaminoglycans are selected among heparan sulfate, dermatan sulfate, chondroitin sulfate A and C, and hyaluronic acid.

3. Use according to claim 1, characterized in that said compositions are in form of ovuli, creams, pommades, lotions and ointments, containing 0.5 to 5% of active component.

4. Use according to claim 3, characterized in that the content of glycosamynoglycans is 1%.

5. Use according to claim 1, characterized in that the resulting composition is used for the prevention and therapy of dystrophy and atrophy states induced by lack of estrogenic hormones, of seniles vaginitis, of compromission and damaging of the vaginal ecosystem, of vaginal dermatitis, of aspecific vaginitis and of aspecific cervicitis.

## Patentansprüche

1. Verwendung von Glykosaminoglykanen - sowohl einzeln als auch in Mischung - als einziger aktiver Bestandteil zur Herstellung einer topischen pharmazeutischen Zusammensetzung zur Prävention und Therapie von Erkrankungen im cervikalen Vaginalbereich.

2. Verwendung nach Anspruch 1, dadurch gekennzeichnet, daß die genannten Glykosaminoglykane ausgewählt werden aus: Heparansulfat, Dermatansulfat, Chondroitinsulfat A und C und Hyaluronsäure.

3. Verwendung nach Anspruch 1, dadurch gekennzeichnet, daß die genannten Zusammensetzungen in Form von Ovuli, Cremes, Pomaden, Lotionen und Salben vorliegen, die 0,5 bis 5% der aktiven Komponente enthalten.

4. Verwendung nach Anspruch 3, dadurch gekennzeichnet, daß der Gehalt an Glkykosaminoglykanen 1% beträgt.

5. Verwendung nach Anspruch 1, dadurch gekenzeichnet, daß die hergestellte Zusammensetzung zur Prävention und Therapie von dystrophischen und atrophischen Zuständen, induziert durch das Fehlen von Östrogenhormonen, von seniler Vaginitis, von Beeinträchtigung bzw. Zerstörung des vaginalen Ökosystems und von vaginaler Dermatitits, von unspezifischer Vaginitis und von unspezifischer Cervicitis verwendet wird.

## Revendications

1. Utilisation de glycosaminoglycanes, à la fois seul et en mélange, à titre de seul ingrédient actif pour la préparation d'une composition pharmaceutique locale, pour la prévention et la thérapie des maladies de la zone vaginale cervicale.

2. Utilisation selon la revendication 1, caractérisée en ce que lesdits glycosaminoglycanes sont sélectionnés parmi le sulfate d'héparane, le sulfate de dermatane, le sulfate de chondroïtine A et C et l'acide hyaluronique.

3. Utilisation selon la revendication 1, caractérisée en ce que lesdites compositions se présentent sous la forme d'ovules, de crèmes, de pommades, de lotions et d'onguents, contenant 0,5 à 5 % de composant actif.

4. Utilisation selon la revendication 3, caractérisée en ce que la teneur en glycosaminoglycanes est de 1 %.

5. Utilisation selon la revendication 1, caractérisée en ce que la composition résultante est utilisée pour la prévention et la thérapie d'états de dystrophie et d'atrophie induit par un manque d'hormones oestrogènes, d'une vaginite sénile, et d'une mise en péril et d'un endommagement de l'écosystème vaginal, d'une dermatite vaginale, d'une vaginite aspécifique et d'une cervicite aspécifique.
